# EUROPEAN PATENT SPECIFICATION

(11) **EP 3 998 050 B1**
(45) Date of publication and mention of the grant of the patent: **08.04.2026**
(21) Application number: 20836965.2
(22) Date of filing: 05.06.2020
(51) Int. Cl.: A61F 2/88, A61F 2/89

(54) **STENT AND AFFIXING METHOD FOR ELEMENT WIRES IN STENT**
STENT UND BEFESTIGUNGSVERFAHREN FÜR ELEMENTDRÄHTE IN EINEM STENT
ENDOPROTHÈSE ET PROCÉDÉ DE FIXATION POUR FILS D'ÉLÉMENT DANS UNE ENDOPROTHÈSE

(30) Priority: 09.07.2019 JP 2019127645
(43) Date of publication of application: 18.05.2022
(73) Proprietor: Pentas Inc., Tokyo 150-0043 (JP)
(72) Inventor: NISHIGISHI, Makoto, Tokyo 150-0043 (JP)
(74) Representative: Zimmermann, Tankred Klaus
(86) International application number: PCT/JP2020/022298
(87) International publication number: WO 2021/005931

(56) References cited:
- JP-A- 2009 240 615
- JP-A- 2009 240 615
- JP-A- 2017 522 070
- JP-A- H09 503 952
- US-A1- 2014 172 074

## Description

### TECHNICAL FIELD

The present invention relates to a stent and a stent strand fixing method.

### BACKGROUND ART

The following medical stent has been known. This stent is formed in such a manner that multiple strands are woven in a spiral shape (JP 2012 223 209 A).

US 2014/ 172 074 A1 relates to stents for insertion into bodily lumens that include at least one strand that forms a self-expanding tubular structure. These strands form a plurality of intersections at which one portion of the strand is in proximity to another portion of the same or different strand. At least some of those intersections include a plurality of reinforcement members.

### SUMMARY OF INVENTION

### PROBLEMS TO BE SOLVED BY INVENTION

Generally, strands are not fixed, but are independent of each other at a stent formed by weaving of the strands. Thus, there is a probability that entanglement of both ends of the stent occurs upon production of the stent and the shape of the stent is deformed accordingly. Moreover, in some cases, displacement of the strands occurs in a catheter upon delivery of the stent. Further, in some cases, expansive force is insufficient only by expansion utilizing the elasticity of the strands upon expansion of the stent. However, no study has been typically conducted on the technique of solving these problems.

### SOLUTIONS TO PROBLEMS

According to a first aspect of the present invention, a stent includes: multiple strands woven in a spiral shape, wherein the stent expands by using elasticity of the strands upon expansion, wherein at an intersection of two crossing strands, the two crossing strands are fixed by a fixing material having rubber elasticity, and
at an intersection between crossing two of the strands, the two of the strands are fixed by the fixing material having an elongated shape along a strand positioned on a lower side in a state in which the two strands overlap with each other.

According to a second aspect of the present invention, in the stent according to the first aspect, a strand intersection positioned in a vicinity of each end of the stent is fixed by the fixing material.

According to a third aspect of the present invention, in the stent according to the first aspect, a strand intersection positioned in a vicinity of each end of the stent and a strand intersection positioned at a center portion of the stent are fixed by the fixing material.

According to a fourth aspect of the present invention, in the stent according to the first aspect, all strand intersections are fixed by the fixing material.

According to a fifth aspect of the present invention, in the stent according to any one of the first to fourth aspects, the fixing material is a material having the rubber elasticity, such as resin having a shape-memory property or bioabsorbable resin.

According to a sixth aspect of the present invention, a method for fixing ones of multiple strands of a stent configured such that the multiple strands are woven in a spiral shape, and the stent configured to expand using elasticity of the strands upon expansion, the method including: fixing, by a fixing material having rubber elasticity, and an elongated shape along a strand positioned on a lower side in a state in which the two strands overlap with each other, two crossing strands at an intersection of the two crossing strands.

According to a seventh aspect of the present invention, in the stent strand fixing method according to the sixth aspect, a strand intersection positioned in a vicinity of each end of the stent is fixed by the fixing material.

According to an eighth aspect of the present invention, in the stent strand fixing method according to the sixth aspect, a strand intersection positioned in a vicinity of each end of the stent and a strand intersection positioned at a center portion of the stent are fixed by the fixing material.

According to a ninth aspect of the present invention, in the stent strand fixing method according to the sixth aspect, all strand intersections are fixed by the fixing material.

According to a tenth aspect of the present invention, in the stent strand fixing method according to any one of the sixth to ninth aspects, the fixing material is a material having the rubber elasticity, such as resin having a shape-memory property or bioabsorbable resin.

### EFFECTS OF INVENTION

According to the present invention, the two crossing strands are, at the strand intersection, fixed by the fixing material having the rubber elasticity, and therefore, occurrence of entanglement of both ends of the stent upon production thereof can be prevented. Moreover, occurrence of displacement of the strands in a catheter upon delivery of the stent can also be prevented. Further, even in a case where expansive force is insufficient only by expansion utilizing the elasticity of the strands upon expansion of the stent, expansion of the stent is assisted by the fixing material having the rubber elasticity so that the stent can be uniformly expanded.

### BRIEF DESCRIPTION OF DRAWINGS

Fig. 1 is a view schematically showing the shape of a stent.
Fig. 2 is a view schematically showing a state in which entanglement has occurred at both ends of the stent.
Fig. 3 is a view schematically showing a state in which markers are displaced.
Fig. 4 is a view schematically showing a state in which two crossing strands are fixed at a strand intersection.
Fig. 5 is a view schematically showing a fixed portion using a fixing material.
Fig. 6 is a view showing a variation of the strand fixing portions.
Fig. 7 is a first view showing a variation of a strand fixing method.
Fig. 8 is a second view showing a variation of the strand fixing method.
Fig. 9 is a third view showing a variation of the strand fixing method.

### DESCRIPTION OF EMBODIMENTS

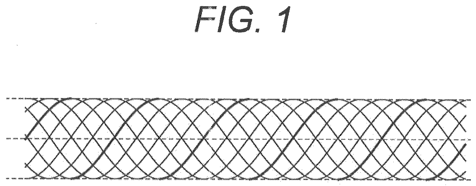

Fig. 1 is a view schematically showing the shape of a stent. In the present embodiment, a stent is configured such that multiple strands are woven in a spiral shape as shown in Fig. 1. As the strand, a strand made of metal such as stainless steel, cobalt-chromium alloy (Co-Cr alloy), or nickel-titanium alloy (Ni-Ti alloy) is used, for example.

The stent is formed in such a manner that the multiple metal strands are woven in the spiral shape as described above, and there are multiple types of the number of strands forming the stent. Examples include a stent having a 16-strand structure in which 16 strands are woven, a stent having a 24-strand structure in which 24 strands are woven, and a stent having a 32-strand structure in which 32 strands are woven. Note that Fig. 1 shows the stent having the 16-strand structure.

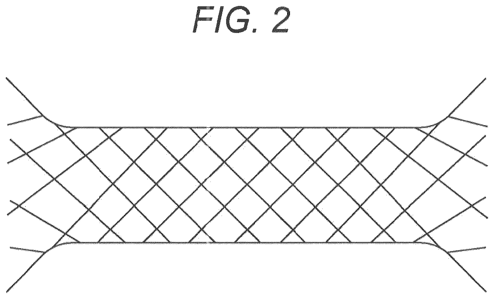

Generally, in the stent formed in such a manner that the multiple metal strands are woven in the spiral shape as described above, both end sides of the strands are not fixed and are independent of each other. Thus, as shown in Fig. 2, there is a probability that entanglement is caused on both these end sides upon production of the stent. When the stent in which entanglement is caused on both end sides as described above, the shape of the stent upon expansion thereof is deformed.

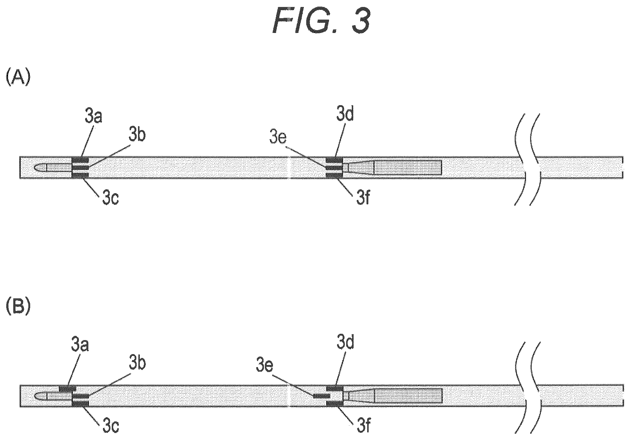

Moreover, displacement of the strands is caused in a catheter upon delivery of the stent in some cases, and in this case, there is a probability that markers fixed to both end sides of the strands are displaced. For example, markers 3a to 3f are not displaced as shown in Fig. 3(A) in a normal state, and on the other hand, the marker 3a and the marker 3e are displaced due to displacement of the strands in an example shown in Fig. 3(B).

Further, in some cases, expansive force is insufficient only by expansion utilizing the elasticity of the strands upon expansion of the stent. That is, at a center portion (an intermediate portion) of the stent, the expansive force is also applied from both sides in addition to the expansive force of the strands at such a center portion, and therefore, sufficient expansive force can be obtained. On the other hand, at both end portions of the stent, the expansive force is applied from a center side, but no expansive force is applied from end sides. Thus, sufficient expansive force is not obtained, and therefore, there is a probability that a problem such as insufficient expansion or nonuniform expansion of the end portions of the stent is caused.

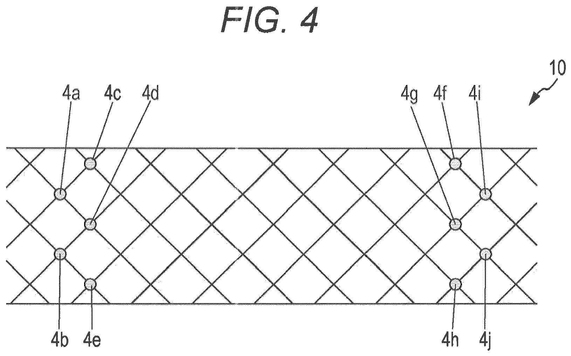

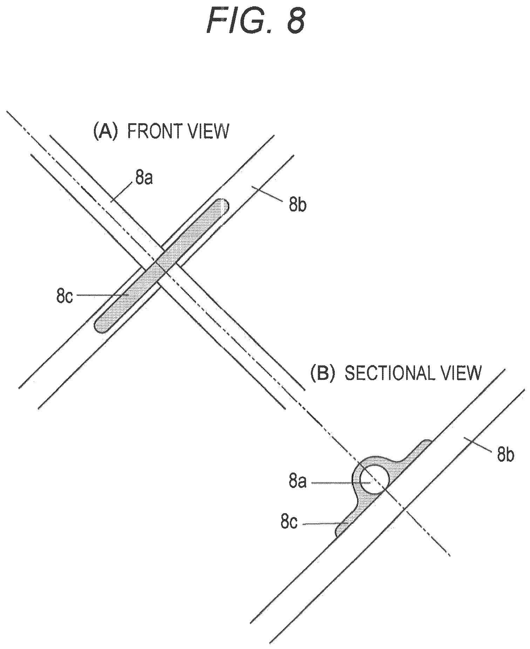

For solving these problems, a stent 10 is, in the present embodiment, configured such that two crossing strands are fixed at an intersection of or between the strands as shown in Fig. 4 and Fig. 8, wherein the embodiment of Fig. 4 is not covered by the present invention. Note that Fig. 4 is a view schematically showing a state in which the strand intersections are fixed using fixing materials 4a to 4j at the expanded stent and shows a state in which the vicinity of both ends of the stent 10 is targeted for fixing of the intersections. Note that in the case of fixing the intersections positioned in the vicinity of both ends of the stent 10, as shown in Fig. 4, intersections positioned at second and third columns from the end of the stent 10 may be fixed.

As described above, the intersections in the vicinity of both ends of the stent 10 are fixed, and therefore, entanglement of both ends of the stent as shown in Fig. 2 can be prevented. Moreover, occurrence of displacement of the strands in the catheter upon stent delivery can be also prevented, and therefore, displacement of the markers as shown in Fig. 3 can also be prevented.

Further, in the stent 10 of the present embodiment, the fixing materials 4a to 4j made of a material having rubber elasticity are used as fixing members for fixing the strand intersections. As the fixing material having the rubber elasticity, resin having shape-memory properties or bioabsorbable resin, such as silicon, may be used, for example. Thus, the fixing materials can contract the stent and deform the material, and can improve the expandability of the stent by means of restoring force provided by the rubber elasticity upon stent expansion.

Specifically, as shown in Fig. 4 not covered by the present invention, the intersections in the vicinity of both ends of the stent 10 are fixed by the fixing materials 4a to 4j having the rubber elasticity, and therefore, the expandability of both stent end portions at which sufficient expansive force might not be able to be obtained only by the expansive force of the strands can be improved.

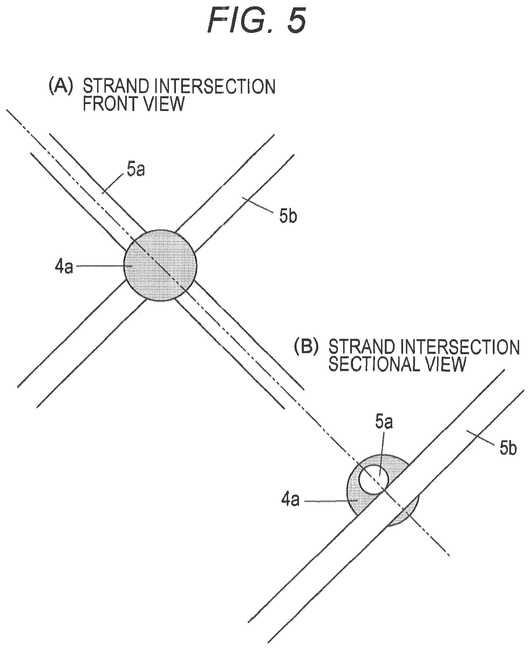

Fig. 5 not covered by the present invention shows schematic views of a state in which an intersection between two strands is fixed using the fixing material 4a. As shown in Fig. 5, at an intersection between a strand 5a and a strand 5b, these two strands are covered with the fixing material 4a in a state in which the strand 5a and the strand 5b overlap with each other, and in this manner, these two strands are fixed. In Fig. 5, Fig. 5(A) is a front view of the strand intersection, and this figure shows that the two strands are fixed by the fixing material 4a at the intersection between the strand 5a and the strand 5b. Moreover, Fig. 5(B) is a sectional view of the strand intersection, and this figure shows that the two strands are covered with the fixing material 4a in a state in which the strand 5a and the strand 5b overlap with each other.

According to the present embodiment described above, the following features and advantageous effects can be obtained.
(1) In the stent configured such that the multiple strands are woven in the spiral shape, the two crossing strands are, at the intersection between the strands, fixed by the fixing material having the rubber elasticity. With this configuration, entanglement of both stent ends can be prevented. Moreover, occurrence of displacement of the strands in the catheter upon stent delivery can be also prevented. Further, the strand intersection is fixed by the fixing material having the rubber elasticity. Thus, the fixing material can contract the stent and deform the material, and can improve the expandability of the stent by means of the restoring force provided by the rubber elasticity upon stent expansion.
(2) The strand intersections positioned in the vicinity of both ends of the stent are fixed with the fixing materials. With this configuration, the expandability of both stent end portions at which sufficient expansive force might not be able to be obtained only by the expansive force of the strands can be improved.

### Variations

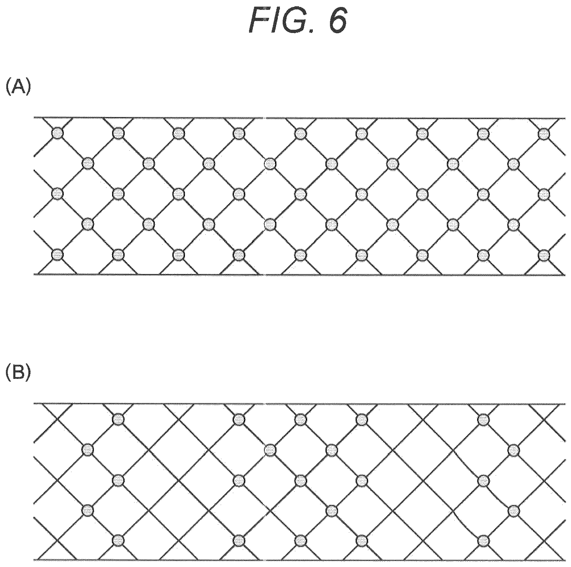

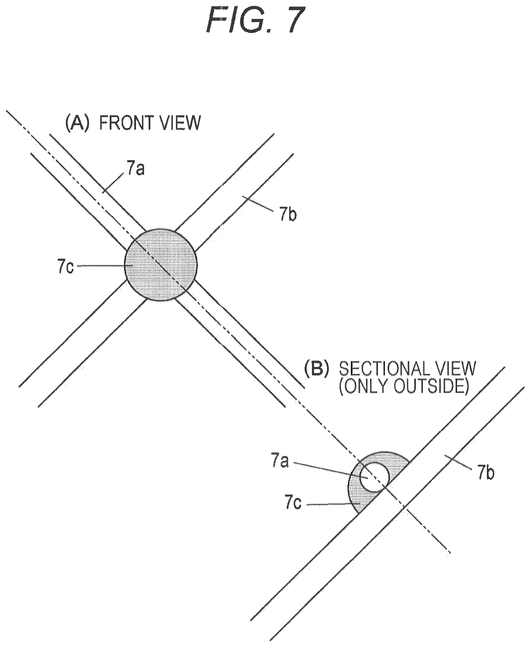

Note that the stent of the above-described embodiment can be modified as follows.
(1) In the above-described embodiment, the example where the intersections positioned at the second and third columns from the end of the stent 10 are fixed as shown in Fig. 4, i.e., the intersections in the vicinity of both ends of the stent 10 are fixed, has been described. However, as long as the object of the present invention can be achieved, the position of the intersection at which the strands are fixed is not limited to the vicinity of both ends. For example, as shown in Fig. 6(A) not covered by the present invention, all strand intersections may be fixed by fixing materials. Alternatively, as shown in Fig. 6(B) not covered by the present invention, strand intersections positioned in the vicinity of both ends of the stent and strand intersections positioned at the center portion of the stent may be fixed by fixing materials. Since the strand intersections are fixed in any of the examples shown in Figs. 6(A) and 6(B), entanglement of both stent ends and displacement of the strands in the catheter upon stent delivery can be prevented as in the above-described embodiment. Moreover, since the strands are fixed at the intersections close to both ends of the stent in any of these examples, the expandability of both stent ends at which the expansive force tends to be insufficient can be improved.
(2) In the above-described embodiment, the example where the two strands are covered and fixed with the fixing material in a state in which the two strands overlap with each other as shown in Fig. 5 has been described. However, the method for fixing the strands by the fixing material is not limited to such a method. For example, as shown in Fig. 7 not covered by the present invention, at an intersection between a strand 7a and a strand 7b, only a front side (a stent outer side) of these two strands may be fixed by a hemispherical fixing material 7c in a state in which the strand 7a and the strand 7b overlap with each other. In Fig. 7, Fig. 7(A) is a front view of the strand intersection, and this figure shows that the two strands are fixed by the fixing material 7c at the intersection between the strand 7a and the strand 7b. Moreover, Fig. 7(B) is a sectional view of the strand intersection, and this figure shows that the outside, i.e., the front side, of the two strands is fixed by the hemispherical fixing material 7c in a state in which the strand 7a and the strand 7b overlap with each other.

According to the present invention as shown in Fig. 8, at an intersection between a strand 8a and a strand 8b, these two strands may be fixed by an elongated fixing material 8c along the strand 8b positioned on the lower side in a state in which the strand 8a and the strand 8b overlap with each other. In Fig. 8, Fig. 8(A) is a front view of the strand intersection, and this figure shows that the two strands are fixed by the elongated fixing material 8c along the strand 8b at the intersection between the strand 8a and the strand 8b. Moreover, Fig. 8(B) is a sectional view of the strand intersection, and this figure shows that the outside of the two strands is fixed by the fixing material 8c in a state in which the strand 8a and the strand 8b overlap with each other.

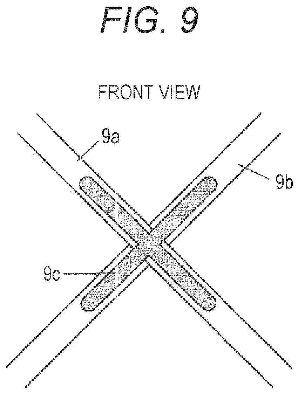

Alternatively, as shown in Fig. 9 covered by the present invention, at an intersection between a strand 9a and a strand 9b, these two strands may be fixed by a cross-shaped fixing material 9c along the strand 9a and the strand 9b in a state in which the strand 9a and the strand 9b overlap with each other. In any method, the above-described object of the present invention can be achieved using the material having the rubber elasticity as the fixing material.

Moreover, the above-described embodiment and multiple variations may be combined with each other.

### LIST OF REFERENCE SIGNS

10 Stent
3a to 3f Marker
4a to 4j, 7c, 8c, 9c Fixing Material
5a, 5b, 7a, 7b, 8a, 8b, 9a, 9b Strand

## Claims

1. A stent (10) comprising:
multiple strands (5a, 5b, 7a, 7b, 8a, 8b, 9a, 9b ) woven in a spiral shape,
wherein the stent (10) expands by using elasticity of the strands (5a, 5b, 7a, 7b, 8a, 8b, 9a, 9b ) upon expansion, wherein
at an intersection of two crossing strands (5a, 5b, 7a, 7b, 8a, 8b, 9a, 9b ), the two crossing strands (5a, 5b, 7a, 7b, 8a, 8b, 9a, 9b ) are fixed by a fixing material (4a to 4j, 7c, 8c, 9c ) having rubber elasticity, and
an elongated shape along a strand (5a, 5b, 7a, 7b, 8a, 8b, 9a, 9b ) positioned on a lower side in a state in which the two strands (5a, 5b, 7a, 7b, 8a, 8b, 9a, 9b ) overlap with each other.

2. The stent (10) according to claim 1, wherein
a strand intersection positioned in a vicinity of each end of the stent (10) is fixed by the fixing material (4a to 4j, 7c, 8c, 9c ).

3. The stent (10) according to claim 1, wherein
a strand intersection positioned in a vicinity of each end of the stent (10) and a strand intersection positioned at a center portion of the stent (10) are fixed by the fixing material (4a to 4j, 7c, 8c, 9c ).

4. The stent (10) according to claim 1, wherein
all strand intersections are fixed by the fixing material (4a to 4j, 7c, 8c, 9c ).

5. The stent (10) according to any one of claims 1 to 4, wherein
the fixing material (4a to 4j, 7c, 8c, 9c ) comprises a resin having a shape-memory property or a bioabsorbable resin.

6. A method for fixing ones of multiple strands (5a, 5b, 7a, 7b, 8a, 8b, 9a, 9b ) of a stent (10) configured such that the multiple strands (5a, 5b, 7a, 7b, 8a, 8b, 9a, 9b ) are woven in a spiral shape, and the stent (10) configured to expand using elasticity of the strands (5a, 5b, 7a, 7b, 8a, 8b, 9a, 9b ) upon expansion, comprising:
fixing, by a fixing material (4a to 4j, 7c, 8c, 9c ) having rubber elasticity, and an elongated shape along a strand (5a, 5b, 7a, 7b, 8a, 8b, 9a, 9b ) positioned on a lower side in a state in which the two strands (5a, 5b, 7a, 7b, 8a, 8b, 9a, 9b ) overlap with each other, two crossing strands (5a, 5b, 7a, 7b, 8a, 8b, 9a, 9b ) at an intersection of the two crossing strands (5a, 5b, 7a, 7b, 8a, 8b, 9a, 9b ).

7. The stent strand fixing method according to claim 6, wherein
a strand intersection positioned in a vicinity of each end of the stent (10) is fixed by the fixing material (4a to 4j, 7c, 8c, 9c ).

8. The stent strand fixing method according to claim 6, wherein
a strand intersection positioned in a vicinity of each end of the stent (10) and a strand intersection positioned at a center portion of the stent (10) are fixed by the fixing material (4a to 4j, 7c, 8c, 9c ).

9. The stent strand fixing method according to claim 6, wherein
all strand intersections are fixed by the fixing material (4a to 4j, 7c, 8c, 9c ).

10. The stent strand fixing method according to any one of claims 6 to 9, wherein
the fixing material (4a to 4j, 7c, 8c, 9c ) comprises a resin having a shape-memory property or a bioabsorbable resin.

## Patentansprüche

1. Ein Stent (10), der folgende Merkmale aufweist:
mehrere Stränge (5a, 5b, 7a, 7b, 8a, 8b, 9a, 9b), die spiralförmig verwoben sind,
wobei sich der Stent (10) durch Verwendung von Elastizität der Stränge (5a, 5b, 7a, 7b, 8a, 8b, 9a, 9b) bei einer Ausdehnung ausdehnt, wobei
an einem Schnittpunkt von zwei sich überkreuzenden Strängen (5a, 5b, 7a, 7b, 8a, 8b, 9a, 9b) die zwei sich überkreuzenden Stränge (5a, 5b, 7a, 7b, 8a, 8b, 9a, 9b) durch ein Befestigungsmaterial (4a bis 4j, 7c, 8c, 9c) befestigt sind, das eine Gummielastizität hat, und
eine längliche Form entlang eines Strangs (5a, 5b, 7a, 7b, 8a, 8b, 9a, 9b) aufweist, der in einem Zustand, in dem sich die zwei Stränge (5a, 5b, 7a, 7b, 8a, 8b, 9a, 9b) überlappen, auf einer unteren Seite positioniert ist.

2. Der Stent (10) gemäß Anspruch 1, wobei
ein Strangschnittpunkt, der in einer Nähe jedes Endes des Stents (10) positioniert ist, durch das Befestigungsmaterial (4a bis 4j, 7c, 8c, 9c) befestigt ist.

3. Der Stent (10) gemäß Anspruch 1, wobei
ein Strangschnittpunkt, der in einer Nähe jedes Endes des Stents (10) positioniert ist, und ein Strangschnittpunkt, der in einem mittleren Abschnitt des Stents (10) positioniert ist, durch das Befestigungsmaterial (4a bis 4j, 7c, 8c, 9c) befestigt sind.

4. Der Stent (10) gemäß Anspruch 1, wobei
alle Strangschnittpunkte durch das Befestigungsmaterial (4a bis 4j, 7c, 8c, 9c) befestigt sind.

5. Der Stent (10) gemäß einem der Ansprüche 1 bis 4, wobei
das Befestigungsmaterial (4a bis 4j, 7c, 8c, 9c) ein Harz mit einer Formgedächtniseigenschaft oder ein bioabsorbierbares Harz aufweist.

6. Ein Verfahren zum Befestigen einzelner von mehreren Strängen (5a, 5b, 7a, 7b, 8a, 8b, 9a, 9b) eines Stents (10), der so konfiguriert ist, dass die mehreren Drähte (5a, 5b, 7a, 7b, 8a, 8b, 9a, 9b) spiralförmig verwoben sind, und wobei der Stent (10) dazu konfiguriert ist, sich durch Verwendung von Elastizität der Stränge (5a, 5b, 7a, 7b, 8a, 8b, 9a, 9b) bei einer Ausdehnung auszudehnen, das folgende Schritte aufweist:
Befestigen, durch ein Befestigungsmaterial (4a bis 4j, 7c, 8c, 9c), das Gummielastizität und eine längliche Form entlang eines Stranges (5a, 5b, 7a, 7b, 8a, 8b, 9a, 9b) aufweist, der in einem Zustand, in dem sich die beiden Drähte (5a, 5b, 7a, 7b, 8a, 8b, 9a, 9b) überlappen, an einer unteren Seite positioniert ist, von zwei sich überkreuzenden Strängen (5a, 5b, 7a, 7b, 8a, 8b, 9a, 9b) an einem Schnittpunkt der beiden sich überkreuzenden Stränge (5a, 5b, 7a, 7b, 8a, 8b, 9a, 9b).

7. Das Stentdrahtbefestigungsverfahren gemäß Anspruch 6, wobei
ein Strangschnittpunkt, der in einer Nähe jedes Endes des Stents (10) positioniert ist, durch das Befestigungsmaterial (4a bis 4j, 7c, 8c, 9c) befestigt ist.

8. Das Stentdrahtbefestigungsverfahren gemäß Anspruch 6, wobei
ein Strangschnittpunkt, der in einer Nähe jedes Endes des Stents (10) positioniert ist, und ein Draht-Schnittpunkt, der in einem mittleren Abschnitt des Stents (10) positioniert ist, durch das Befestigungsmaterial (4a bis 4j, 7c, 8c, 9c) befestigt sind.

9. Das Stentdrahtbefestigungsverfahren gemäß Anspruch 6, wobei
alle Strangschnittpunkte durch das Befestigungsmaterial (4a bis 4j, 7c, 8c, 9c) befestigt sind.

10. Das Stentdrahtbefestigungsverfahren gemäß einem der Ansprüche 6 bis 9, wobei das Befestigungsmaterial (4a bis 4j, 7c, 8c, 9c) ein Harz mit einer Formgedächtniseigenschaft oder ein bioabsorbierbares Harz aufweist.

## Revendications

1. Stent (10) comprenant :
de multiples brins (5a, 5b, 7a, 7b, 8a, 8b, 9a, 9b) tissés en forme de spirale,
dans lequel le stent (10) se dilate en utilisant l'élasticité des brins (5a, 5b, 7a, 7b, 8a, 8b, 9a, 9b) lors de la dilatation, dans lequel
à une intersection de deux brins croisés (5a, 5b, 7a, 7b, 8a, 8b, 9a, 9b), les deux brins croisés (5a, 5b, 7a, 7b, 8a, 8b, 9a, 9b) sont fixés par un matériau de fixation (4a à 4j, 7c, 8c, 9c) ayant une élasticité de caoutchouc, et
une forme allongée le long d'un brin (5a, 5b, 7a, 7b, 8a, 8b, 9a, 9b) positionné sur un côté inférieur dans un état où les deux brins (5a, 5b, 7a, 7b, 8a, 8b, 9a, 9b) se chevauchent l'un l'autre.

2. Stent (10) selon la revendication 1, dans lequel
une intersection de brins positionnée à proximité de chaque extrémité du stent (10) est fixée par le matériau de fixation (4a à 4j, 7c, 8c, 9c).

3. Stent (10) selon la revendication 1, dans lequel
une intersection de brins positionnée à proximité de chaque extrémité du stent (10) et une intersection de brins positionnée dans une partie centrale du stent (10) sont fixées par le matériau de fixation (4a à 4j, 7c, 8c, 9c).

4. Stent (10) selon la revendication 1, dans lequel
toutes les intersections de brins sont fixées par le matériau de fixation (4a à 4j, 7c, 8c, 9c).

5. Stent (10) selon l'une quelconque des revendications 1 à 4, dans lequel
le matériau de fixation (4a à 4j, 7c, 8c, 9c) comprend une résine ayant une propriété de mémoire de forme ou une résine bioabsorbable.

6. Procédé pour fixer certains des multiples brins (5a, 5b, 7a, 7b, 8a, 8b, 9a, 9b) d'un stent (10) configuré de telle sorte que les multiples brins (5a, 5b, 7a, 7b, 8a, 8b, 9a, 9b) soient tissés en forme de spirale, et le stent (10) soit configuré pour se dilater en utilisant l'élasticité des brins (5a, 5b, 7a, 7b, 8a, 8b, 9a, 9b) lors de l'expansion, comprenant :
la fixation, à l'aide d'un matériau de fixation (4a à 4j, 7c, 8c, 9c) présentant une élasticité caoutchouteuse et une forme allongée le long d'un brin (5a, 5b, 7a, 7b, 8a, 8b, 9a, 9b) positionné sur un côté inférieur dans un état dans lequel les deux brins (5a, 5b, 7a, 7b, 8a, 8b, 9a, 9b) se chevauchent l'un l'autre, deux brins croisés (5a, 5b, 7a, 7b, 8a, 8b, 9a, 9b) à une intersection des deux brins croisés (5a, 5b, 7a, 7b, 8a, 8b, 9a, 9b).

7. Procédé de fixation d'un fil de stent selon la revendication 6, dans lequel
une intersection de brins située à proximité de chaque extrémité du stent (10) est fixée par le matériau de fixation (4a à 4j, 7c, 8c, 9c).

8. Procédé de fixation d'un fil de stent selon la revendication 6, dans lequel
une intersection de brins positionnée à proximité de chaque extrémité du stent (10) et une intersection de brins située dans une partie centrale du stent (10) sont fixées par le matériau de fixation (4a à 4j, 7c, 8c, 9c).

9. Procédé de fixation d'un fil de stent selon la revendication 6, dans lequel
toutes les intersections de brins sont fixées par le matériau de fixation (4a à 4j, 7c, 8c, 9c).

10. Procédé de fixation d'un fil de stent selon l'une quelconque des revendications 6 à 9, dans lequel
le matériau de fixation (4a à 4j, 7c, 8c, 9c) comprend une résine ayant une propriété de mémoire de forme ou une résine bioabsorbable.
